# EUROPÄISCHE PATENTSCHRIFT

(11) **EP 2 036 972 B1**
(45) Veröffentlichungstag und Bekanntmachung des Hinweises auf die Patenterteilung: **29.02.2012**
(21) Anmeldenummer: 07018144.1
(22) Anmeldetag: 14.09.2007
(51) Int. Cl.: C11D 1/14, C11D 3/20, C11D 1/94, C11D 1/65, A61K 8/46, A61K 8/34, C11D 11/04, C11D 11/00, C11D 1/90, C11D 1/52

(54) **Hochkonzentrierte Fettalkoholsulfatzubereitung**
Highly concentrated fatty alcohol sulphate compound
Préparation de sulfate d'alcools gras hautement concentrée

(43) Veröffentlichungstag der Anmeldung: 18.03.2009
(73) Patentinhaber: Cognis IP Management GmbH, 40589 Düsseldorf (DE)
(72) Erfinder: Spörer, Roland, Klongtoey Bangkok 10110 (TH); Jariyachat, Srarat, Chonburi 20000 (TH); Waworuntu, Tedy, Bogor 16968 (ID); Jazuli, Achmad, Blok DD2/10-RT 2 /RW X, Ciomas-Bogor 16610 Jawa Barat (ID); Taufik, Achmad, Mampang Depok 16433 (ID)
(74) Vertreter: Reinhardt, Jürgen

(56) Entgegenhaltungen:
- EP-A- 0 554 991
- WO-A-94/24242
- WO-A-96/35770
- WO-A-97/38972
- WO-A-2006/112492
- DE-A1- 4 420 515
- US-A- 5 536 332

## Beschreibung

Die vorliegende Anmeldung betrifft konzentrierte Tensidzubereitungen auf Basis von Alkylsulfat-Tensiden, deren Verwendung und deren Herstellung.

Fettalkoholsulfate sind bekannte anionische Tenside, die in einer Vielzahl von Wasch- und Reinigungsmitteln, aber auch in kosmetischen Mitteln, z.B. in Shampoos, Hautreinigungsmitteln, Hautseifen aber auch in Zahnpasten Verwendung finden.

Die Alkylsulfate werden großtechnisch hergestellt, in der Regel durch Sulfierung geeigneter Fettalkohole mit gasförmigem SO₃. Die dabei als Rohprodukte anfallenende Alkylschwefelsäure wird anschließend in Gegenwart geeigneter Alkalien, vorzugsweise von wässerigem Natriumhydroxid zu den Alkylsulfaten neutralisiert. Am Ende solcher Prozesse werden Tensidhaltige wässerige Zubereitungen mit maximal 30 Gew.-% an Anionentensiden erhalten.

Zum Transport solcher Zubereitungen wird häufig das Wasser entfernt, um trockene Produkte zu erhallten, die leichter zu transportieren sind. Die Entfernung des Wassers stellt aber einen viel Energie konsumierenden und somit teuren Verfahrensschritt dar. Auch der Transport der direkt anfallenden wässerigen Mittel selbst ist nicht vorteilhaft, werden doch so nur vergleichsweise geringe Mengen an Aktivsubstanz (maximal 30 Gew.-%) aber viel Wasser transportiert. Außerdem ist die Verarbeitung von getrockeneten Fettalkoholsulfaten zeitaufwendiger. Der Einsatz von fluessigen Fettalkoholsulfaten ergibt in der Regel eine Batchzeitreduzierung von mehr als 20% auf der Seite der Hersteller von Shampoos, Hautreinigungsmitteln, Hautseifen aber auch in Zahnpasten.

Es besteht daher ein Bedarf, pumpfähige, flüssige bzw. leicht pastöse, hoch konzentrierte Fettalkoholsulfat-Zubereitungen bereit zu stellen, um die oben geschilderten Probleme zu vermeiden. Aus der WO 2006/112492 A sind Zusammensetzungen bekannt, die anionische Tenside, ausgewählt aus der Gruppe Alkylsulfate oder Alkylethersulfate in Kombination mit z.B. Betainen und Wasser enthalten, und die trotzdem noch fließfähig sind. Die EP 554 991 offenbart eine wässrige, flüssige oder pastöse Reinigungsmittelzusammensetzung, die Alkylsulfat und Betaine nebeneinander enthält. Aus der DE 44 20 515 A1 sind Rasiercremes offenbart, die neben Alkylpolyglycosiden noch Aniontenside und Wasser sowie Glycerin enthalten, wobei der Anteil an Aniontensiden beispielhaft bei max. 28 Gew.-% liegt. Der Wassergehalt der Beispielrezepturen liegt bei max. 40 Gew.-%.

Es wurde gefunden, dass man höher konzentrierte, pumpfähige, also niedrigviskose Fettalkoholzubereitungen durch Zusatz bestimmter Additive erhalten kann.

Ein erster Gegenstand der Anmeldung betrifft daher Mittel ein Mittel, dass als Flüssigkeit oder niedrig viskose pumpfähige Paste vorliegt, enthaltend mindestens
(a) 35 Gew.-%, bezogen auf das Gesamtgewicht des Mittels, eines oder mehrerer Fettalkoholsulfate der allgemeinen Formel (I)

   R¹O-SO₃X (I)

   in der R¹ für einen linearen oder verzweigten, aliphatischen Alkyl- und/oder Alkenylrest mit 6 bis 22, vorzugsweise 12 bis 18 Kohlenstoffatomen und X für ein Alkali- und/oder Erdalkalimetall, Ammonium, Alkylammonium, Alkanolammonium oder Glucammonium steht,
(b) mindestens ein Polyol ausgewählt aus der Gruppe, Glycerin, Ethylenglykol, Propylenglykol, oder Sorbitol
(c) Wasser.

Unter Alkyl- und/oder Alkenylsulfaten, die auch häufig als "Fettalkoholsulfate" bezeichnet werden, sind die Sulfatierungsprodukte primärer Alkohole zu verstehen, die der Formel (I) folgen,

R¹O-SO₃X (I)

in der R¹ für einen linearen oder verzweigten, aliphatischen Alkyl- und/oder Alkenylrest mit 6 bis 22, vorzugsweise 12 bis 18 Kohlenstoffatomen und X für ein Alkali- und/oder Erdalkalimetall, Ammonium, Alkylammonium, Alkanolammonium oder Glucammonium steht. Typische Beispiele für Alkylsulfate, die im Sinne der Erfindung Anwendung finden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelen'schen Oxosynthese erhalten werden. Die Sulfatierungsprodukte können vorzugsweise in Form ihrer Alkalisalze und insbesondere ihrer Natriumsalze eingesetzt werden.

Besonders bevorzugt sind Alkylsulfate auf Basis von C_{16/18}-Talgfettalkoholen bzw. pflanzliche Fettalkohole vergleichbarer C-Kettenverteilung in Form ihrer Natriumsalze. Von besonderer Bedeutung können Alkylsulfate aus Basis von Dodecylalkohol sein, da diese Sulfate in Zahlpflege- und Zahnreinigungsmitteln Verwendung finden. Weiterhin bevorzugt sind aber auch Fettalkoholschnitte aus Fettalkoholen mit 12 bis 14 C-Atomen, wobei es hierbei besonders bevorzugt sein kann solche Schnitte zu verwenden, die nur sehr geringe Anteile an C16-Fettalkoholen enthalten, z.B. weniger als 10 Gew.-%, vorzugsweise weniger als 5 Gew.% und insbesondere weniger als 3 Gew.-%, bezogen auf die Menge an Fettalkoholen. Besonders bevorzugt sind im Weiteren die gesättigten Fettalkohole und hier wiederum die oben bezeichneten Schnitte.

Die erfindungsgemäßen Mittel sind dadurch ausgezeichnete, das sie hochkonzentriert sind, d.h. im Zusammenhang dieser Anmeldung dass sie mindestens 35 Gew.-% an Fettalkoholsulfaten, bezogen auf die gesamte Zusammensetzung enthalten. Die Mittel gemäß der beanspruchten technischen Lehre können aber vorzugsweise Fettalkoholsulfate in Mengen, bezogen auf das Gesamtgewicht des Mittels, von mehr als 35 Gew.-%, vorzugsweise von 36 Gew.-% bis 70 Gew.%, insbesondere 40 bis 60 Gew.-% und besonders bevorzugt von 40 bis 50 Gew.-% enthalten.

Erfindungswesentlich ist die Gegenwart von Polyolen, oder den Betainen bzw. den Fettsäurealkylamiden, wobei aber aus dieser Dreiergruppe die Polyole die bevorzugte Auswahl darstellen.

Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind Glycerin; Alkylenglycole, wie beispielsweise Ethylenglycol, Di-ethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin; Dialkoholamine, wie Diethanolamin oder 2-Amino-1,3-propandiol. Bevorzugte Polyole sind ausgewählt aus der Gruppe Glycerin, Ethylenglykol, Propylenglykol, oder Sorbitol, wobei Glycerin und Sorbitol besonders bevorzugt ist.

Betaine stellen bekannte Tenside dar, die überwiegend durch Carboxyalkylierung, vorzugsweise Carboxymethylierung von aminischen Verbindungen hergestellt werden. Vorzugsweise werden die Ausgangsstoffe mit Halogencarbonsäuren oder deren Salzen, insbesondere mit Natriumchloracetat kondensiert, wobei pro Mol Betain ein Mol Salz gebildet wird. Ferner ist auch die Anlagerung von ungesättigten Carbonsäuren, wie beispielsweise Acrylsäure möglich. Besonders bevorzugt sind Betaine, die durch Umsetzung von Ölen, also Triglyceriden erhalten werden. Als Beispiel sei das N,N-Dimethyl-N(kokosamidopropyl)ammoniumacetobetain (Dehyton PK 45 - Fa. Cognis) genannt. Bevorzugt sind öllösliche Betaine.

Fettsäurealkyamide sind ebenfalls an sich bekannte Verbindungen. Darunter werden auch Fettsäurealkanolamide im engeren Sinne verstanden wobei hier die Fettsäuredi- oder monoethanolamide besonders bevorzugt sind. Dabei sind weiterhin Verbindungen dieses Typs, enthaltend gesättigte oder ungesättigte, verzweigte oder unverzweigte Fettsäuren mit C-Kettenlängen von 7 bis 21 bevorzugt. Diese werden beispielsweise durch die Anmelderin unter der Marke "Comperlan" vertrieben. Besonders bevorzugt sind Fettsäurealkylamide auf Basis von Kokosfettsäuremonoethanolamiden. Es gilt, dass öllösliche Fettsäurealkylamide besonders bevorzugt sind.

Die Polyole aber auch die Betaine bzw. die Fettsäurealkylamide sind in den Mitteln in Mengen, bezogen auf das Gesamtgewicht der Mittel, von 10 bis 45 Gew.-%, vorzugsweise 15 bis 40 und insbesondere 25 bis 35 Gew.-% enthalten. Es kann bevorzugt sein, Mischungen von Polyolen, Betainen und/oder Fettsäurealkylamiden zu verwenden.

Die erfindungsgemäßen Mittel enthalten zwingend die Fettalkoholsulfate, Polyole oder Betaine oder die Fettsäurealkylamide und Wasser, können darüber hinaus aber noch andere Inhaltsstoffe enthalten, z.B. anorganische Salze oder nichtionische Tenside. Besonders bevorzugt sind aber Mittel, die nur aus den drei genannten Komponenten bestehen.

Die wässerigen Mittel weisen vorzugsweise einen pH-Wert von größer 8 auf, vorzugsweise liegt der pH-Wert im Bereich von 9 bis 12, besonders bevorzugt im Bereich von 10 bis 12. Die Mittel können als Flüssigkeit oder als Paste, vorzugsweise als niedrig viskose, pumpfähige Paste vorliegen. Typische Viskositäten, gemessen nach Brookfield bei 25 °C (Spindel 3 oder 4, 10 rpm) liegen bei 500 bis 10000 mPas und vorzugsweise bei 1000 bis 8000 mPas.

Ein bevorzugtes Mittel im Sinne der vorliegenden Erfindung enthält bezogen auf das Gewicht des gesamten Mittels enthält (a) 36 bis 45 Gew.-% an Alkylsulfaten der Formel (I), (b) 28 bis 45 Gew.-% an Polyolen, wobei Glycerin und/oder Sorbitol bevorzugt sind, oder Betainen und/oder Fettsäurealkylamiden und (c) 10 bis 34 Gew.-% an Wasser.

Die Herstellung der konzentrierten Fettalkoholzubereitungen erfolgt vorzugsweise durch Neutralisation der schwefelsauren Fettalkoholester in Gegenwart eines Wasser/Polyol bzw. Betain-Gemisches. Ein weiterer Gegenstand betrifft daher ein Verfahren zur Herstellung einer Zubereitung, enthaltend mindestens 35 Gew.-% an Alkylsulfaten der Formel (I) gemäß der obigen Beschreibung, wobei kontinuierlich zu einer Mischung aus (A) Wasser und (B) einem Polyol oder Betain - wobei das Volumenverhältnis von (A) zu (B) eingestellt wird auf Werte von 3 : 1 bis 1 : 3 - ein nicht-neutralisiertes Alkylsulfat der allgemeinen Formel R¹-OSO₃H zusammen mit einer Menge an Alkalien zugegeben wird, wobei die Menge an Alkalien geeignete ist das Alkylsulfat vollständig zu neutralisieren und das Vermischen der Mischung aus (A) und (B) mit dem nicht-neutralisierten Alkylsulfat und den Alkalien solange fortgesetzt wird, bis mindestens ein Konzentration an neutralisierten Alkylsulfaten der Formel (I) von 35 Gew.-% erreicht worden ist.

Die Sulfierung der Fettalkohole erfolgt in an sich bekannter Art und Weise mit SO₃. Das rohe Sulfierprodukt wird dann zu der Mischung aus (A) und (B) zudosiert und gleichzeitig wird vorzugsweise separat eine wässerige Alkalilauge, vorzugsweise wässeriges Natriumhydroxid zudosiert, so dass eine gleichzeitige Vermischung und Neutralisation stattfindet. Diese Vorgehensweise wird solange durchgeführt, bis der gewünschte Konzentrationsbereich der Fettalkoholsulfatzubereitung erreicht wird. Danach können, sofern gewünscht noch weitere Additive oder Inhaltsstoffe zudosiert werden. Das Verfahrensprodukt kann dann z.B. aber auch noch gebleicht und/oder mit Duftstoffen versehen. Es kann vorteilhaft sein, das Mengenverhältnis von (A) und (B) untereinander zu variieren, wobei ein Volumenverhältnis im Bereich von 3 : 1 bis 1 : 1 und insbesondere von 2 : 1 bis 1,5 : 1 besonders bevorzugt sein kann.

### Beispiele

Es wurden eine Fettalkoholmischung mit der qualitativen Zusammensetzung C12: 65-71 Gew.-%; C14: 22-28 Gew.-%; C16: 3-6 Gew.-% in einer SO₃-Anlage sulfiert. Das sulfierte Rohprodukt wurde dann kontinuierlich zu einer Mischung aus Polyole mit Wasser im Gewichtsverhältnis von 3 : 1 zusammen mit einer 30-%igen wässerigen NaOH-Lösung in einem Edelstahlneutralisationsbehälter zudosiert. Das Endprodukt nach erfolgter Neutralisation wurde ausgeschleust. Es wies einen Gehalt an sulfierten Fettalkoholen von 42 Gew.-% auf. Der pH-Wert betrug 11. Die Farbe (APHA, 20 Gew.-%ige Lösung) wurde mit 50 gemessen.

Es wurden auf diese Weise Produkte (Fettalkoholsulfat gemäß der oben beschriebenen qualitativen Zusammensetzung des Fettalkoholgemisches) mit den folgenden Zusammensetzungen (alle Angaben in Gew.%) hergestellt:

| **Nr.** | **Fettalkoholsulfat** | **Glycerin** | **Sorbitol** | **Betain¹⁾** | **Fettsäurealkylamid** ²⁾ | **Wasser** |
|---|---|---|---|---|---|---|
| 1 | 30 | 40 | | | | 60 |
| 2 | 40 | 26 | 19 | | | 85 |
| 3 | 35 | 26 | | 10 | | 29 |
| 4 | 35 | 26 | | | 10 | 29 |

| | | | | | | |
|---|---|---|---|---|---|---|
| 1) Dehyton PK 45 2) Comperlan CMEA | | | | | | |

Die Beispiele 1, 3 und 4 sind Vergleichsbeispiele, wohingegen das Beispiel 2 erfindungsgemäß ist.

## Patentansprüche

1. Mittel, das als Flüssigkeit oder niedrig viskose pumpfähige Paste vorliegt, enthaltend mindestens
(a) 35 Gew.-%, bezogen auf das Gesamtgewicht des Mittels, eines oder mehrerer Fettalkoholsulfate der allgemeinen Formel (I)
R¹O-SO₃X (I)
in der R¹ für einen linearen oder verzweigten, aliphatischen Alkyl- und/oder Alkenylrest mit 6 bis 22, vorzugsweise 12 bis 18 Kohlenstoffatomen und X für ein Alkali- und/oder Erdalkalimetall, Ammonium, Alkylammonium, Alkanolammonium oder Glucammonium steht,
(b) mindestens ein Polyol ausgewählt aus der Gruppe, Glycerin, Ethylenglykol, Propylenglykol, oder Sorbitol und
(c) Wasser.

2. Mittel nach Anspruch 1, **dadurch gekennzeichnet, dass** es Fettalkoholsulfate in Mengen, bezogen auf das Gesamtgewicht des Mittels, von mehr als 35 Gew.-%, vorzugsweise von 36 Gew.-% bis 70 Gew.-%, insbesondere 40 bis 60 Gew.-% und besonders bevorzugt von 40 bis 50 Gew.-% enthält.

3. Mittel nach mindestens einem der Ansprüche 1 bis 2, **dadurch gekennzeichnet, dass** als Polyol Glycerin und/oder Sorbitol besonders bevorzugt sind.

4. Mittel nach mindestens einem der Ansprüche 1 bis 3, **dadurch gekennzeichnet, dass** es die Komponente b) in Mengen, bezogen auf das Gesamtgewicht des Mittels, von 10 bis 45 Gew.-%, vorzugsweise 15 bis 40 und insbesondere 25 bis 35 Gew.-% enthält.

5. Mittel nach mindestens einem der Ansprüche 1 bis 4, **dadurch gekennzeichnet, dass** es Wasser in Mengen, bezogen auf das Gesamtgewicht des Mittels, von 5 bis 40, vorzugsweise 10 bis 30 und insbesondere von 15 bis 25 Gew.-% enthält.

6. Mittel nach mindestens einem der Ansprüche 1 bis 5, **dadurch gekennzeichnet, dass** es, bezogen auf das Gewicht des gesamten Mittels enthält
(a) 36 bis 45 Gew.-% an Alkylsulfaten der Formel (I)
(b) 28 bis 45 Gew.-% an Polyolen, Betainen und/oder Fettsäurealkylamiden
(c) 10 bis 34 Gew.-% an Wasser

7. Verfahren zur Herstellung einer Zubereitung, enthaltend mindestens 35 Gew.-% an Alkylsulfaten der Formel (I) gemäß der Beschreibung im Anspruch 1, **dadurch gekennzeichnet, dass** kontinuierlich zu in einer Mischung aus (A) Wasser und (B) einem Polyol oder öllöslichen Betain - wobei das Volumenverhältnis von (A) zu (B) eingestellt wird auf Werte von 3 : 1 bis 1 : 3 - ein nicht-neutralisiertes Alkylsulfat der allgemeinen Formel R¹-OSO₃H zusammen mit einer Menge an Alkalien zugegeben wird, wobei die Menge an Alkalien geeignete ist das Alkylsulfat vollständig zu neutralisieren und das Vermischen der Mischung aus (A) und (B) mit dem nicht-neutralisierten Alkylsulfat und den Alkalien solange fortgesetzt wird, bis mindestens ein Konzentration an neutralisierten Alkylsulfaten der Formel (I) von 35 Gew.-% erreicht worden ist.

## Claims

1. Composition which is in the form of a liquid or low-viscosity pumpable paste, comprising at least
(a) 35% by weight, based on the total weight of the composition, of one or more fatty alcohol sulfates of the general formula (I)
R¹O-SO₃X (I)
in which R¹ is a linear or branched, aliphatic alkyl and/or alkenyl radical having 6 to 22, preferably 12 to 18, carbon atoms and X is an alkali metal and/or alkaline earth metal, ammonium, alkylammonium, alkanolammonium or glucammonium,
(b) at least one polyol selected from the group glycerol, ethylene glycol, propylene glycol or sorbitol and
(c) water.

2. Composition according to Claim 1, **characterized in that** it comprises fatty alcohol sulfates in amounts, based on the total weight of the composition, of more than 35% by weight, preferably of from 36% by weight to 70% by weight, in particular 40 to 60% by weight and particularly preferably from 40 to 50% by weight.

3. Composition according to at least one of Claims 1 to 2, **characterized in that** glycerol and/or sorbitol are particularly preferred as polyol.

4. Composition according to at least one of Claims 1 to 3, **characterized in that** it comprises component b) in amounts, based on the total weight of the composition, of from 10 to 45% by weight, preferably 15 to 40% by weight and in particular from 25 to 35% by weight.

5. Composition according to at least one of Claims 1 to 4, **characterized in that** it comprises water in amounts, based on the total weight of the composition, of from 5 to 40% by weight, preferably 10 to 30% by weight and in particular from 15 to 25% by weight.

6. Composition according to at least one of Claims 1 to 5, **characterized in that** it comprises, based on the weight of the total composition,
(a) 36 to 45% by weight of alkyl sulfates of the formula (I),
(b) 28 to 45% by weight of polyols, betaines and/or fatty acid alkylamides,
(c) 10 to 34% by weight of water.

7. Method for producing a preparation, comprising at least 35% by weight of alkyl sulfates of the formula (I) according to the description in Claim 1, **characterized in that** an unneutralized alkyl sulfate of the general formula R¹-OSO₃H is continuously added, together with an amount of alkalis, to a mixture of (A) water and (B) a polyol or oil-soluble betaine - where the volume ratio of (A) to (B) is adjusted to values of from 3:1 to 1:3 - where the amount of alkalis is suitable to completely neutralize the alkyl sulfate, and the mixing of the mixture of (A) and (B) with the unneutralized alkyl sulfate and the alkalis is continued until at least a concentration of neutralized alkyl sulfates of the formula (I) of 35% by weight has been reached.

## Revendications

1. Agent, se trouvant sous forme liquide ou de pâte pouvant être pompée, de basse viscosité, contenant au moins
(a) 35% en poids, par rapport au poids total de l'agent, d'un ou de plusieurs sulfates d'alcools gras de formule générale (I)
R¹O-SO₃X (I)
dans laquelle R¹ représente un radical alkyle et/ou alcényle linéaire ou ramifié, aliphatique, comprenant 6 à 22, de préférence 12 à 18 atomes de carbone et X représente un métal alcalin et/ou alcalino-terreux, ammonium, alkylammonium, alcanolammonium ou glucammonium,
(b) au moins un polyol choisi dans le groupe formé par le glycérol, l'éthylèneglycol, le propylèneglycol ou le sorbitol et
(c) de l'eau.

2. Agent selon la revendication 1, **caractérisé en ce qu'**il contient les sulfates d'alcools gras en des quantités, par rapport au poids total de l'agent, supérieures à 35% en poids, de préférence de 36% en poids à 70% en poids, en particulier de 40 à 60% en poids et de manière particulièrement préférée de 40 à 50% en poids.

3. Agent selon au moins l'une quelconque des revendications 1 à 2, **caractérisé en ce qu'**on préfère en particulier, comme polyol, le glycérol et/ou le sorbitol.

4. Agent selon au moins l'une quelconque des revendications 1 à 3, **caractérisé en ce qu'**il contient le composant b) en des quantités, par rapport au poids total de l'agent, de 10 à 45% en poids, de préférence de 15 à 40 et en particulier de 25 à 35% en poids.

5. Agent selon au moins l'une quelconque des revendications 1 à 4, **caractérisé en ce qu'**il contient l'eau en des quantités, par rapport au poids total de l'agent, de 5 à 40 en poids, de préférence de 10 à 30 et en particulier de 15 à 25% en poids.

6. Agent selon au moins l'une quelconque des revendications 1 à 5, **caractérisé en ce qu'**il contient, par rapport au poids de la totalité de l'agent
(a) 36 à 45% en poids d'alkylsulfates de formule (I)
(b) 28 à 45% en poids de polyols, de bétaïnes et/ou d'alkylamides d'acides gras
(c) 10 à 34% en poids d'eau.

7. Procédé pour la préparation d'une composition contenant au moins 35% en poids d'alkylsulfates de formule (I) selon la description dans la revendication 1, **caractérisé en ce qu'**on ajoute en continu, à un mélange de (A) de l'eau et de (B) un polyol ou une bétaïne soluble dans l'huile - le rapport volumique de (A) à (B) étant réglé à des valeurs de 3:1 à 1:3 - un alkylsulfate non neutralisé de formule générale R¹-OSO₃H ensemble avec une quantité d'alcalis, la quantité d'alcalis étant appropriée pour neutraliser complètement l'alkylsulfate et le mélange du mélange de (A) et de (B) avec l'alkylsulfate non neutralisé et les alcalis étant continué jusqu'à ce qu'on atteigne une concentration en alkylsulfates neutralisés de formule (I) d'au moins 35% en poids.
